# EUROPÄISCHE PATENTSCHRIFT

(11) **EP 0 588 106 B1**
(45) Veröffentlichungstag und Bekanntmachung des Hinweises auf die Patenterteilung: **24.01.1996**
(21) Anmeldenummer: 93113408.4
(22) Anmeldetag: 23.08.1993
(51) Int. Cl.: C07C 17/26

(54) **Verfahren zur Herstellung von Hexafluorpropen-Dimeren mit einem hohen Anteil an Perfluor-(4-methyl-2-penten)**
Process for the preparation of hexafluoropropene dimers with a high content of perfluoro-(4-methyl-2-pentene)
Procédé de préparation de dimeres d'hexa fluoropropène ayant un taux élevé de perfluoro-(4-méthyl-2-pentène)

(30) Priorität: 27.08.1992 DE 4228592
(43) Veröffentlichungstag der Anmeldung: 23.03.1994
(73) Patentinhaber: HOECHST AKTIENGESELLSCHAFT, D-65926 Frankfurt am Main (DE)
(72) Erfinder: Gisser, Alfons, D-84508 Burgkirchen (DE); von Werner, Konrad Dr., D-84518 Garching (DE)

(56) Entgegenhaltungen:
- US-A- 2 918 501
- US-A- 3 917 724
- JOURNAL OF ORGANIC CHEMISTRY, Bd. 30, Nr. 10 , Oktober 1965, Easton, US, Seiten 3524-3527, R. D. DRESDNER et al, "Alkali Fluorides as Catalysts in Reactions Involving Unsaturated Fluorocarbons"

## Beschreibung

Bei der bekannten Dimerisierung von Hexafluorpropen wird teilweise in erheblichem Ausmaß das toxische Perfluor-(2-methyl-2-penten) neben wechselnden Anteilen an trimerem Hexafluorpropen gebildet. Es ist bekannt, aus dem Rohgemisch der Destillation die Dimeren von den Trimeren zu trennen und beispielsweise ein Gemisch von 98 % trans-Perfluor-(4-methyl-2-penten), im folgenden (I), neben 2 % Perfluor-(2-methyl-2-penten), im folgenden (II), zu isolieren (US-A 2 918 501, Beispiel V). Bei diesem bekannten Verfahren isoliert man jedoch die etwa 4fache Menge an Trimeren neben den Dimeren.

Die Dimeren des Hexafluorpropens eignen sich bei vielen Einsatzgebieten als Ersatz für Chlorfluorkohlenwasserstoffe, die für den Abbau des Ozons in der Stratosphäre verantwortlich gemacht werden. Erhebliche Mengen an (II) wären natürlich aufgrund der Toxizität in einem Substitutionsprodukt unerwünscht. Es bestand deshalb die Aufgabe, Dimere des Hexafluorpropens mit einem möglichst hohen Anteil an (I) und einem möglichst niedrigen Anteil an (II) herzustellen, wobei möglichst wenig an den hier unerwünschten Trimeren gebildet werden sollen.

Diese Aufgabe wird erfindungsgemäß gelöst, indem man Hexafluorpropen in Gegenwart eines Addukts aus einem Amin, welches keine N-H-Gruppe enthält, und einem Metallfluorid in einem aprotischen Lösemittel dimerisiert.

Überraschenderweise werden hierbei ganz überwiegend Dimere mit einem hohen Gehalt an (I) und wenig (II) gebildet.

Aus Halasz et al., Chem. Ber. 106 (1973) 2950 bis 2955, war es bekannt, aus Triethanolamin und Hexafluorpropen zunächst tris-[2-(2H-Hexafluorpropoxy)ethyl]amin herzustellen und mit diesem in Acetonitril die Oligomerisierung von Hexafluorpropen zu katalysieren. Hierbei werden zu 90,5 % Dimere gebildet, aus denen 96 % (I) und 3,9 % (II) isoliert werden konnten.

Aus der US-A 4 042 638 ist ein Verfahren zur Herstellung von Di- und Trimeren von Hexafluorpropen bekannt, bei dem man Hexafluorpropen in Gegenwart eines Komplexsalzes eines Alkalimetallhalogenids mit einem Kronenether oligomerisiert. Man erhält mit so einem Katalysator aus Kaliumfluorid und 1,4,7,10,13,16-Hexaoxacyclooctadecan 87,2 % Oligomere mit 99,1 % Dimeren, wobei das Dimere 95,8 % (I) und 4,2 % eines Gemisches der entsprechenden cis-Verbindung und (II) enthielt.

Alle diese bekannten Verfahren sind umständlich und hinsichtlich der Ausbeute unbefriedigend. Bei dem erfindungsgemäßen Verfahren wird demgegenüber die Verbindung (I) in einfacher Weise und hoher Ausbeute mit einem so niedrigen Gehalt an (II) erhalten, daß das Rohprodukt unmittelbar einsatzfähig ist.

Der Ablauf des erfindungsgemäßen Verfahrens muß als überraschend angesehen werden, da Alkalifluoride als Katalysatoren bei der Dimerisierung von Hexafluorpropen als Hauptprodukt (II) bilden können [Dresdner et al., J. Org. Chem. 30 (1965) 3524 bis 3526]. Weiterhin war es bekannt (Brunskill et al., Chem. Comm. 1970, 1444 bis 1446), daß das Fluoridion die Umlagerung von (I) in (II) katalysiert.

Es war zwar aus der vorstehend genannten US-A 2 918 501 bekannt, daß man Hexafluorpropen mit Haliden, beispielsweise Fluoriden und Lösungsmitteln aus der Klasse der alkylsubstituierten Amide, Phenylamine und Sulfoxide umsetzen kann, wobei Dimere und Trimere erhalten werden. Die höchste Ausbeute an Dimeren wird dabei mit Dimethylanilin in Methanol erhalten, wobei aber immer noch über 10 % an Trimeren erhalten werden.

Allen diesen bekannten Verfahren konnte somit kein Anhaltspunkt entnommen werden, daß das erfindungsgemäß eingesetzte Katalysatorsystem sehr selektiv die Verbindung (I) bildet.

Im folgenden werden bevorzugte Ausgestaltungen der Erfindung näher erläutert.

Als Metallfluorid wird bevorzugt Kaliumfluorid eingesetzt. Als Amin-Komponente werden bevorzugt peralkylierte, chelatbildende Diamine oder Triamine verwendet, die in den Alkyl- oder Alkylengruppen auch Ethersauerstoff enthalten können. Das bevorzugte Verhältnis von Amin-Stickstoffatomen zu Metallatom im Metallfluorid beträgt 1 : 1 bis 4 : 1. Die Komplexe aus Amin und Metallfluorid werden am einfachsten durch Vermischen der Komponenten im Lösungsmittel vor der Umsetzung mit Hexafluorpropen (HFP) hergestellt.

Bevorzugte Lösungsmittel sind Nitrile, zum Beispiel Acetonitril, Propionitril oder Benzonitril. Cyclische oder offenkettige Ether, beispielsweise Tetrahydrofuran, 1,4-Dioxan oder Diethylenglykoldimethylether ergeben schlechtere Resultate.

Alle Reagenzien sollen möglichst wasserfrei sein, um die Bildung von H-haltigen Verunreinigungen zu vermeiden.

Die Umsetzung der Katalysatorlösung mit dem gasförmig zudosierten HFP wird am einfachsten in einem Rührbehälter durchgeführt. Die Reaktionstemperatur beträgt vorzugsweise 35 bis 60 °C. Nach Beendigung der Umsetzung trennt sich das Reaktionsgemisch in zwei Phasen. Das sich als schwerere Phase abscheidende rohe HFP-Dimer wird abgelassen. Die im Reaktor verbleibende Katalysatorlösung kann für die nächste Charge verwendet werden.

Das Rohprodukt wird einer Wasserwäsche unterzogen, um es von Resten des Katalysators und Lösungsmittels zu befreien. Diese Reinigungsoperation wird bevorzugt im Gegenstrom durchgeführt. Nach Abtrennung des Waschwassers wird das Rohprodukt durch Destillation über eine Kolonne mit einer gut gekühlten Vorlage gereinigt.

Eine typische Herstellung ist im Beispiel 1 beschrieben. Die tabellierten Beispiele 2 bis 9 wurden mit anderen Katalysatorsystemen durchgeführt.

Das erfindungsgemäß erhaltene Perfluorisohexen ist wie FCKW 113 unbrennbar und hat somit gegenüber den Ölen und Glykolen, die ebenfalls als FCKW-Substitute eingesetzt werden, sicherheitstechnische Vorteile. Seine Zündtemperatur ist mit 420 °C so hoch, daß sie bei den üblichen Anwendungstemperaturen nicht zu einer Gefahr werden kann.

Das erfindungsgemäß hergestellte Perfluorisohexen ist eine reine Fluor-Kohlenstoff-Verbindung, ist also im Gegensatz zu FCKW 113 chlorfrei und beeinflußt somit die Ozonschicht in der Stratosphäre nicht. Sein Ozonabbaupotential (ODP) ist gleich Null. Wegen seiner relativ reaktiven Doppelbindung ist seine Lebensdauer in der Atmosphäre geringer als die der gesättigten Perfluoralkane, die ebenfalls als FCKW-113-Substitute in Frage kommen. Wegen seiner geringen akuten Säugetier-(oral), Bakterien- und Fischtoxizität, jedoch schlechten biologischen Abbaubarkeit, ist das erfindungsgemäß erhältliche Perfluorisohexen als nur schwach wassergefährdend einzustufen.

### Beispiel 1

### Herstellung von Hexafluorpropen-Dimeren:

Diese Versuche erfolgten in einem 16-l-Kessel aus elektropoliertem Edelstahl, der mit einem regelbaren Rührer, einer geeichten Zuführung für gasförmiges Hexafluorpropen sowie Dosierungen für Flüssigkeiten und Feststoffe ausgerüstet ist. Heizung und Kühlung des Kessels kann über einen Kreislauf mit einem externen Platten-Wärmetauscher auf ± 1 °C genau geregelt werden.

Zunächst wird im Labor eine Katalysatorlösung aus folgenden Komponenten hergestellt:
- 1,5 l Acetonitril (entwässert durch Destillation über etwas Calciumhydrid und Aufbewahren über 3-Å-Molekularsieb),
- 69,7 g (0,6 mol) N,N,N',N'-Tetramethylethylendiamin (getrocknet über 4-Å-Molekularsieb),
- 34,9 g (0,6 mol) Kaliumfluorid-Pulver (getrocknet durch Rühren des Pulvers bei 180 °C/0,5 mbar).

Die Komponenten werden unter trockenem Stickstoff in dieser Reihenfolge bei laufendem Rührer zusammengegeben und bei 30 bis 40 °C 1 Stunde intensiv gerührt. Man erhält eine gelbliche Lösung, die kaum noch festes Kaliumfluorid enthält.

Diese Lösung wird in den sorgfältig mit N₂ gespülten 16-l-Kessel eingefüllt. Man heizt den Kessel bei geschlossenem Kopfventil auf 40 °C auf und dosiert dann innerhalb von 6 Stunden bei laufendem Rührer 16 kg gasförmiges HFP zu. Die Reaktion ist leicht exotherm. Die Innentemperatur wird durch Kühlung bei 40 bis 50 °C gehalten. Danach läßt man noch 2 Stunden bei 40 °C nachreagieren und kühlt dann die Mischung bei abgestelltem Rührer auf 15 °C ab.

Über das Bodenventil des Kessels werden langsam 14 kg Rohprodukt in einen Behälter abgelassen, der zum Druckausgleich mit der HFP-Zuleitung zum Kessel verbunden ist (Pendelleitung). 2 kg des Produktes verbleiben mit der leichteren Katalysatorphase im Kessel.

Das Rohprodukt wird durch Gaschromatographie analysiert. Zur Analyse wird eine gepackte Säule (5 % GEXE 60 auf Porasil C) mit einem Wärmeleitfähigkeits-Detektor verwendet (Trägergas: Helium). Die Zuordnung der Komponenten wurde vorher durch Bestimmung der Retentionszeiten und Flächenfaktoren mit weitgehend reinen Referenzverbindungen ermittelt. Man erhält folgende Verteilung der fluorhaltigen Produkte:

| | |
|---|---|
| CF₃-CF=CF₂ | 0,2 % |
| (CF₃)₂CFH | 0,1 % |
| trans-(CF₃)₂CF-CF=CF-CF₃ (I) | 90,1 % |
| cis-(CF₃)₂CF-CF=CF-CF₃ | 5,0 % |
| (CF₃)₂C=CF-CF₂CF₃ (II) | 0,4 % |
| Σ HFP-Trimere | 4,2 % |

Durch ¹⁹F-NMR-Analyse des Rohprodukts (Lösung in CDCl₃/R113, Standard: CF₃CO₂H) wurde bestätigt, daß das durch GC bestimmte Verhältnis von trans- und cis-HFP-Dimeren dem Molverhältnis dieser beiden Verbindungen entspricht. Dies wurde durch die Auswertung der Integrale der an die Doppelbindung gebundenen Fluoratome ermittelt. Nachfolgend sind die ¹⁹F-NMR-Kenndaten der beiden Isomeren aufgeführt (Standard: Trifluoressigsäure). 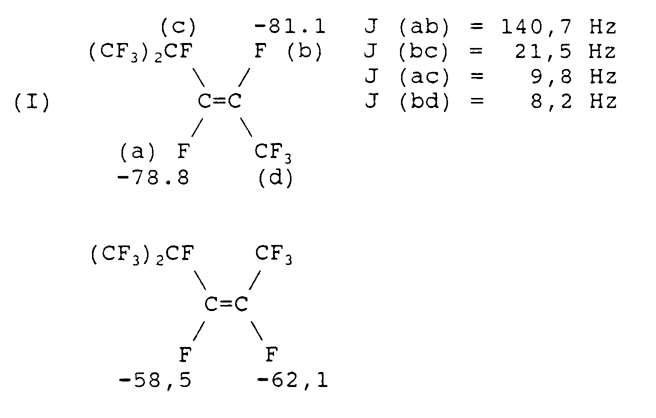
Mit der im Kessel verbliebenen Katalysatorlösung wurden noch zwei weitere Chargen durchgeführt, wobei jeweils 14 kg HFP umgesetzt wurden. Nach der insgesamt dritten Charge wurde die Katalysatorlösung vom Produkt abgetrennt.

Die GC-Analyse ergab folgende Produktverteilungen:

| | Charge 2 | Charge 3 |
|---|---|---|
| CF₃CF=CF₂ | 0,5 % | 1,0 % |
| (CF₃)₂CFH | 0,1 % | 0,2 % |
| trans-(CF₃)₂CF-CF=CF-CF₃ (I) | 90,1 % | 90,3 % |
| cis-(CF₃)₂CF-CF=CF-CF₃ | 5,1 % | 5,0 % |
| (CF₃)₂C=CF-CF₂CF₃ (II) | 0,4 % | 0,2 % |
| (CF₃)₂CH-CF₂CF₂CF₃ | 0,1 % | 0,1 % |
| Σ HFP-Trimere | 3,7 % | 3,2 % |

Das hochtoxische Perfluor-2-methyl-1-penten konnte in keiner Charge nachgewiesen werden.

Insgesamt wurden durch Vereinigung der Rohprodukte 43,5 kg erhalten. Dieses Material enthielt noch Spuren von Verunreinigungen, die aus der Katalysatorlösung stammen. Es wurde durch Gegenstromwasche in einer mit Raschigringen gefüllten vertikalen Blasensäule gereinigt. Die Säule war mit 10 l Wasser gefüllt. Das Fluorprodukt wurde am Kopf zudosiert und über die Säule verrieselt. Gleichzeitig wurden im Gegenstrom 20 l Wasser am unteren Ende eingebracht. Das gereinigte Produkt sammelt sich am unteren Ende der Säule in einem Auffanggefäß. Nach Abtrennen einer überstehenden Wasserschicht enthielt das Produkt (43 kg) weniger als 0,005 % Wasser.

Zur abschließenden Reinigung wurde das gewaschene Produkt bei Normaldruck über eine Füllkörperkolonne destilliert, deren Kondenser und Auffangbehälter mit Hilfe eines Kühlaggregates auf 0 °C gekühlt wurde. Als Hauptfraktion wurden 40 kg mit einem Siedebereich von 45,5 bis 49,5 °C erhalten. Zusammensetzung nach GC:

| | |
|---|---|
| CF₃-CF=CF₂ | 0,1 % |
| trans-(CF₃)₂CF-CF=CF-CF₃ (I) | 94,1 % |
| cis-(CF₃)₂CF-CF=CF-CF₃ | 4,9 % |
| (CF₃)₂C=CF-CF₂CF₃ (II) | 0,2 % |
| Σ HFP-Trimere | 0,7 % |

Die Masseausbeute beträgt 90 % bezogen auf eingesetztes HFP.

### Beispiele 2 bis 9

Die Komponenten des Katalysatorsystems (Metallfluorid und Amin) wurden mit dem Lösungsmittel in einem Rührkolben unter Schutzgas (Stickstoff oder Argon) 1 Stunde bei 30 bis 40 °C gerührt. Diese Mischung wurde dann unter Schutzgas in einen 300-ml-Rührautoklaven aus Edelstahl gegeben. Bei laufendem Rührer wurden dann 150 g (1,0 mol) Hexafluorpropen in 30 Minuten eingegast und die Mischung wurde noch 1,5 Stunden gerührt. Das Rohprodukt wurde in einem Scheidetrichter von der Katalysatorlösung abgetrennt und mit einer Wasser-Eis-Mischung im Scheidetrichter geschüttelt. Die so erhaltene Produktphase wurde gewogen und per GC analysiert. Die Ergebnisse sind in der folgenden Tabelle dargestellt.

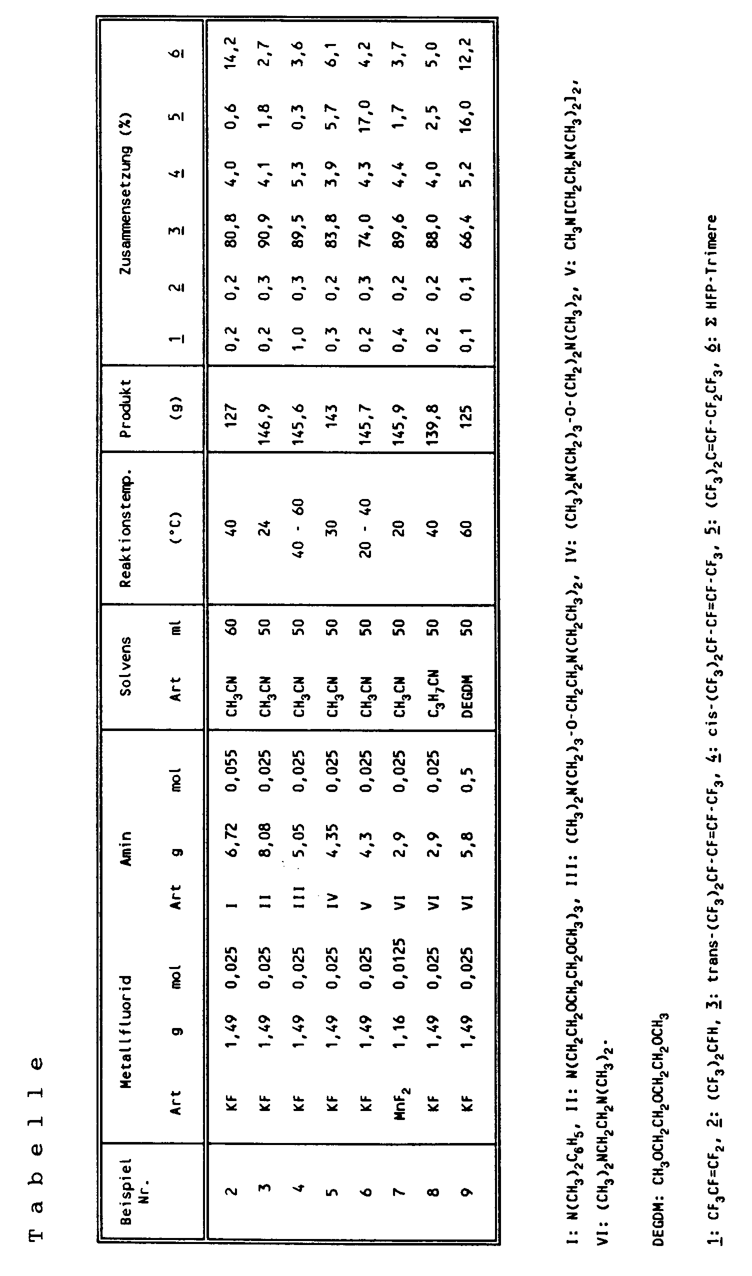

## Patentansprüche

1. Verfahren zur Herstellung von Dimeren des Hexafluorpropens mit einem hohen Anteil an Perfluor-(4-methyl-2-penten) und einem niedrigen Gehalt an Perfluor-(2-methyl-2-penten), dadurch gekennzeichnet, daß die Dimerisierung des Hexafluorpropens in einem aprotischen Lösemittel in Gegenwart eines Adduktes aus einem Amin, welches keine NH-Gruppen enthält, und einem Metallfluorid erfolgt.

2. Verfahren nach Anspruch 1, dadurch gekennzeichnet, daß das Amin pro Stickstoff drei Alkyl- oder Alkylengruppen aufweist, die Ethersauerstoffatome enthalten können.

3. Verfahren nach den Ansprüchen 1 oder 2, dadurch gekennzeichnet, daß für das Addukt Diamine oder Triamine eingesetzt werden.

4. Verfahren nach Anspruch 1, dadurch gekennzeichnet, daß das Verhältnis von Aminstickstoffatomen zum Metallatom des Metallfluorids 1 : 1 bis 4 : 1 beträgt.

5. Verfahren nach Anspruch 1 oder 4, dadurch gekennzeichnet, daß Kaliumfluorid verwendet wird.

6. Verfahren nach Anspruch 1, dadurch gekennzeichnet, daß Acetonitril als Lösemittel verwendet wird.

7. Verfahren nach Anspruch 1, dadurch gekennzeichnet, daß das Addukt aus Amin und Metallfluorid in situ im Lösemittel hergestellt wird.

## Claims

1. A process for the preparation of dimers of hexafluoropropene having a high proportion of perfluoro-(4-methyl-2-pentene) and a low content of perfluoro-(2-methyl-2-pentene), which comprises carrying out the dimerization of the hexafluoropropene in an aprotic solvent in the presence of an adduct of an amine, which does not contain NH groups, and a metal fluoride.

2. The process as claimed in claim 1, wherein the amine, per nitrogen, has three alkyl or alkylene groups which can contain ether oxygen atoms.

3. The process as claimed in either of claims 1 or 2, wherein diamines or triamines are used for the adduct.

4. The process as claimed in claim 1, wherein the ratio of amine nitrogen atoms to the metal atom of the metal fluoride is 1:1 to 4:1.

5. The process as claimed in claim 1 or 4, wherein potassium fluoride is used.

6. The process as claimed in claim 1, wherein acetonitrile is used as solvent.

7. The process as claimed in claim 1, wherein the adduct is prepared from amine and metal fluoride in situ in the solvent.

## Revendications

1. Procédé pour la préparation de dimères de l'hexafluoropropène ayant un taux élevé en perfluoro-(4-méthyl-2-pentène) et un faible taux en perfluoro-(2-méthyl-2-pentène), caractérisé en ce qu'on met en oeuvre la dimérisation de l'hexafluoropropène dans un solvant aprotique en présence d'un produit d'addition d'une amine, qui ne contient pas de groupe NH, et d'un fluorure de métal.

2. Procédé selon la revendication 1, caractérisé en ce que l'amine présente par atome d'azote trois groupes alkyle ou alkylène qui peuvent contenir des atomes d'oxygène d'éther.

3. Procédé selon les revendications 1 ou 2, caractérisé en ce qu'on utilise des diamines ou des triamines pour le produit d'addition.

4. Procédé selon la revendication 1, caractérisé en ce que le rapport des atomes d'azote d'amine à l'atome de métal de fluorure de métal est de 1:1 à 4:1.

5. Procédé selon la revendication 1 ou 4, caractérisé en ce qu'on utilise le fluorure de potassium.

6. Procédé selon la revendication 1, caractérisé en ce qu'on utilise comme solvant l'acétonitrile.

7. Procédé selon la revendication 1, caractérisé en ce qu'on prépare le produit d'addition de l'amine et de fluorure de métal *in situ* dans le solvant.
